(19) Europäisches Patentamt
European Patent Office
Office européen des brevets

(11) **EP 2 090 284 B1**

(12) **FASCICULE DE BREVET EUROPEEN**

(45) Date de publication et mention
de la délivrance du brevet:
**19.12.2012 Bulletin 2012/51**

(51) Int Cl.:
*A61K 8/04* *(2006.01)*          *A61K 8/26* *(2006.01)*
*A61Q 15/00* *(2006.01)*

(21) Numéro de dépôt: **09151386.1**

(22) Date de dépôt: **27.01.2009**

(54) **Composition anti-transpirante a base de microparticules interferentielles ; procede de maquillage et traitement de la transpirationet/ou des odeurs corporelles en particulier axillaires**

Schweisshemmende Zusammensetzung basierend auf Interferenzmikropartikel; Verfahren zum Schminken und zur Behandlung von Schweissabsonderung

Antiperspirant composition based on interference microparticles; makeup method and method for treating perspiration and/or body odours , in particular axillary odours

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **06.02.2008 FR 0850752**

(43) Date de publication de la demande:
**19.08.2009 Bulletin 2009/34**

(73) Titulaire: **L'Oréal**
**75008 Paris (FR)**

(72) Inventeurs:
• **Cassier, Matthieu**
**75017 Paris (FR)**

• **Gliguem, Sandrine**
**95240 CORMEILLES EN PARISIS (FR)**

(74) Mandataire: **Miszputen, Laurent**
**L'Oréal**
**D.I.P.I.**
**25-29 Quai Aulagnier**
**92600 Asnières (FR)**

(56) Documents cités:
**EP-A- 0 972 512          EP-A- 1 433 462**
**EP-A- 1 726 291          EP-A- 1 792 601**
**WO-A-01/78673          DE-A1- 10 141 683**
**US-A1- 2006 246 149**

EP 2 090 284 B1

Printed by Jouve, 75001 PARIS (FR)

**Description**

La présente invention concerne des compositions comprenant dans un milieu cosmétiquement acceptable :

a) un actif anti-transpirant et
b) au moins des particules interférentielles de granulométrie spécifique que l'on détaillera plus loin.

La présente invention a pour objet également un procédé cosmétique de maquillage en particulier de la peau des aisselles et de traitement de la transpiration, consistant à appliquer sur la surface de la peau ladite composition.

La présente invention a pour objet également un procédé cosmétique de maquillage en particulier de la peau des aisselles et de traitement des odeurs corporelles en particulier axillaires en particulier des aisselles, consistant à appliquer sur la surface de la peau une quantité efficace d'une telle composition.

La sueur eccrine ou apocrine est peu odorante lorsqu'elle est sécrétée. C'est sa dégradation par les bactéries via des réactions enzymatiques qui produit des composés malodorants. Les actifs déodorants ont pour fonction de diminuer ou d'empêcher la formation des mauvaises odeurs. Les différents systèmes proposés jusqu'à présent peuvent être regroupés en grandes familles.

(i) Les substances bactéricides ou limitant la croissance des bactéries :
Il y a les substances bactéricides qui détruisent la flore bactérienne résidente. Parmi ces substances, la plus employée est le Triclosan (2,4,4'-trichloro-2'-hydroxydiphényléther). Il y a les substances qui diminuent la croissance des bactéries. Parmi ces substances, on peut citer les chélatants de métaux de transition comme le l'EDTA ou le DPTA.

(ii) Les substances bloquant les réactions enzymatiques responsables de la formation des composés odorants.
En exemples particuliers, on peut citer les inhibiteurs d'arylsulfatase, de 5-Lipoxygenase, d'aminocylase, de β-glucoronidase.

(iii) Les absorbeurs des mauvaises odeurs :
Les absorbeurs d'odeurs « capturent » ou diminuent la volatilité des composés odorants. On peut citer comme absorbeurs d'odeurs, les zéolites, les cyclodextrines. On sait également que certains types de particules solides peuvent être utilisées comme déodorants telles que les silicates d'oxyde métallique de la demande US2005/063928 ; des particules d'oxyde métallique modifiées par un métal de transition dans les demandes US2005084464 et US2005084474, des aluminosilicates comme ceux décrits dans la demande EP1658863, des particules de dérivés de chitosan nanométriques comme celles décrites dans le brevet US6916465.

(iii) Les anti-transpirants :
Les sels d'aluminium et/ou de zirconium sont les plus couramment utilisés comme actif anti-transpirant. Le principe d'action de ces actifs serait de former un gel dans le canal sudoral. Ce gel créerait un bouchon qui boucherait partiellement les pores sudoraux. Le débit sudoral est ainsi diminué. Ces sels d'aluminium ont aussi une efficacité propre car ce sont des antibactériens. Ils jouent donc aussi un rôle direct sur l'efficacité déodorante en réduisant le nombre de bactéries responsables de la dégradation de la sueur.

Ces différents traitements appliqués sur la peau des aisselles ont tendance à provoquer des altérations de la peau se traduisant par des irrégularités, des inhomogénéités du type tâche pigmentaire en particuliers sur les peaux asiatiques, dyschromie ou bien encore points noirs souvent du à la repousse du poil.

A l'heure actuelle, et sur le marché des produits déodorants/antitranspirants, il n'existe pas de solution permettant de masquer ces irrégularités de façon immédiate et remarquable par le consommateur dès l'application tout en conservant un visuel naturel. De plus, il est important d'atteindre cet objectif avec des matériaux qui soient compatibles dans les formulations antitranspirants et qui n'entrainent pas des traces importantes sur les vêtements en contact avec la peau.

On a déjà proposé dans la demande de brevet JP 2000-086446 des compositions à base d'actif déodorant et/ou d'actif antitranspirant destinées à être appliquées sous les aisselles après épilation dans le but de camoufler les marques dues au traitement d'épilation (rasoir, cire, crème dépilatoire), les rougeurs, les points noirs. Ces compositions contiennent des poudres pour le corps comme le talc, des matières colorantes organiques ou minérales.

On connaît également dans la demande EP972512 des compositions de maquillage en particulier pour le visage comprenant un actif anti-transpirant et une silicone élastomère pour améliorer la tenue de la répartition de la couleur après application au cours du temps et procurer au teint une matité durable pendant plusieurs heures. Ces compositions peuvent comprendre des charges destinées à donner du corps ou de la rigidité à la composition et/ou de la douceur, de la matité et de l'uniformité au maquillage. Parmi ces charges, on peut utiliser des particules interférentielles telles que des nacres qui sont des particules irisées qui réfléchissent la lumière comme la nacre naturelle, le mica recouvert d'oxyde de titane, d'oxyde de fer, de pigment naturel ou d'oxychlorure de bismuth ou le mica titane coloré.

Le problème de masquer les irrégularités de la peau dues au traitement des produits anti-transpirants n'est pas évoqué dans ces documents.

Il subsiste donc le besoin de réaliser des produits anti-transpirants et de masquer les altérations de la peau de façon immédiate et remarquable par le consommateur dès l'application et qui ne provoquent pratiquement pas de trace voire pas du tout sur les vêtements en contact avec la peau.

La demanderesse a découvert que l'on pouvait atteindre cet objectif en utilisant des particules interférentielles dont 50% de la population massique a un diamètre inférieur à 40$\mu$m et dont la population massique ne comporte pas de particule de taille supérieure à 80 $\mu$m. La demanderesse a découvert en effet que l'utilisation de nacres dont la distribution de particules comporte des particules de taille supérieure à 80$\mu$m dans des produits antitranspirants conduisait à des phénomènes plus importants de transfert, de tâchage des vêtements.

Cette découverte est la base de cette invention.

La présente invention concerne des compositions comprenant dans un milieu cosmétiquement acceptable

c) au moins un actif anti-transpirant et

d) au moins des particules interférentielles dont 50% de la population massique a un diamètre ($d_{50}$) inférieur à 40$\mu$m et dont la population massique ne comporte pas de particule de taille supérieure à 80 $\mu$m.

La présente invention a pour objet également un procédé cosmétique de maquillage en particulier de la peau des aisselles et de traitement de la transpiration, consistant à appliquer sur la surface de la peau ladite composition.

La présente invention a pour objet également un procédé cosmétique de maquillage en particulier de la peau des aisselles et de traitement des odeurs corporelles en particulier axillaires, consistant à appliquer sur la surface de la peau une quantité efficace d'une telle composition.

La présente invention concerne également l'utilisation de particules interférentielles dont 50% de la population massique a un diamètre ($d_{50}$) inférieur à 40$\mu$m et dont la population massique ne comporte de particule de taille supérieure à 80 $\mu$m dans une composition comprenant dans un milieu cosmétiquement acceptable au moins un actif anti-transpirant dans le but de masquer les irrégularités de la peau après application de ladite composition.

Par "actif anti-transpirant", on entend toute substance qui, à elle seule, a pour effet de diminuer ou limiter le flux sudoral.

Par « cosmétiquement acceptable », on entend compatible avec la peau et/ou ses phanères, qui présente une couleur, une odeur et un toucher agréables et qui ne génère pas d'inconforts inacceptables susceptibles de détourner la consommatrice d'utiliser cette composition.

## Particules interférentielles

Au sens de la présente invention, l'expression "particules interférentielles" désigne toute particule possédant généralement une structure multicouche telle qu'elle permette la création d'un effet de couleur par interférence des rayons lumineux qui diffractent et diffusent différemment selon la nature des couches. Ainsi, ces particules peuvent présenter des couleurs variant selon l'angle d'observation et l'incidence de la lumière.

Au sens de la présente invention, une structure multicouche entend désigner indifféremment une structure formée d'un substrat recouvert d'une unique couche ou une structure formée d'un substrat recouvert d'au moins deux voire de plusieurs couches consécutives.

La structure multicouche peut ainsi comporter une voire au moins deux couches, chaque couche indépendamment ou non de la (ou les) autre(s) couche(s) étant réalisée(s) en au moins un matériau choisi dans le groupe constitué par les matériaux suivants : MgF2, CeF$_3$, ZnS, ZnSe, Si, SiO$_2$, Ge, Te. Fe$_2$O$_3$, Pt. Va, Al$_2$O$_3$, MgO, Y$_2$O$_3$, S$_2$O$_3$, SiO, HfO$_2$, ZrO$_2$, CeO$_2$, Nb$_2$O$_5$, Ta$_2$O$_5$, TiO$_2$, Ag, Al, Au, Cu, Rb, Ti, Ta, W, Zn, MoS$_2$, cryolithe, alliages, polymères et leurs associations.

Généralement, la structure multicouche est de nature inorganique.

Plus particulièrement, les particules interférentielles considérées selon l'invention peuvent être des pigments interférentiels, ou encore des nacres naturelles ou synthétiques, monocouches ou multicouches, en particulier formées d'un substrat naturel à base, entre autres, de mica et qui est recouvert d'une ou plusieurs couches d'oxyde métallique.

Les particules interférentielles selon l'invention sont caractérisées en ce que 50% de la population massique a un diamètre ($d_{50}$) inférieur à 40 $\mu$m, plus particulièrement inférieur à 30 $\mu$m, notamment inférieur à 20 $\mu$m, et en particulier inférieur à 15 $\mu$m, mesurée par un granulomètre laser, tel que par exemple le Mastersizer 2000® de Malvernet ou le B190 +® de Broockhaven Instrument Corporation.

Conviennent tout particulièrement à l'invention, les nacres de type mica/oxyde d'étain/oxyde de titane telles que par exemple celles commercialisées sous les dénominations TIMIRON SILK BLUE®, TIMIRON SILK RED®, TIMIRON SILK GREEN®, TIMIRON SILK GOLD® et TIMIRON SUPER SILK® proposées par la société MERCK et les nacres mica/oxyde de fer/oxyde de titane telles que par exemple les FLAMENCO SATIN BLUE®, FLAMENCO SATIN RED® et FLAMENCO SATIN VIOLET® et FLAMENCO ORANGE 320C proposées par la société ENGELHARD et leurs mélanges.

Il est entendu que le choix de ces particules interférentielles est opéré de manière à être par ailleurs compatible avec les exigences en terme de clarté et de saturation requises pour les compositions selon l'invention. D'une manière générale, ces particules interférentielles sont présentes en quantité suffisante pour obtenir un effet homogène en terme de coloration tout en préservant la carnation naturelle de la peau.

Plus précisément, ces particules peuvent être présentes dans des quantités allant de 0,01 à 10% en poids et de préférence allant de 0,1 à 5% en poids par rapport au poids total de la composition.

**Actifs anti-transpirants**

Les actifs anti-transpirants sont choisis de préférence parmi les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes. De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'hydroxychlorure d'aluminium commercialisé par la société REHEIS sous la dénomination REACH 301 ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF.

On utilisera plus particulièrement le chlorhydrate d'aluminium sous forme activée ou non activée.

Les actifs anti-transpirants peuvent être présents dans la composition selon l'invention à raison de 0,001 à 30% en poids par rapport au poids total de la composition et de préférence à raison de 0,5 à 25% en poids.

La composition selon l'invention a de préférence un pH allant de 3 à 9 selon le support choisi.

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un objet de l'invention, Les compositions anti-transpirantes sont conditionnés dans un dispositif aérosol ou par un flacon pompe ; dans un dispositif muni d'une paroi ajourée telle qu'une grille ; sous forme de bâtonnets (sticks). Elles contiennent à cet égard les ingrédients généralement utilisés dans ce type de produits et bien connus de l'homme de l'art.

Les compositions selon l'invention destinées à l'usage cosmétique peuvent comporter au moins une phase aqueuse. Elle sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse desdites compositions contient de l'eau et en général d'autres solvants solubles ou miscibles dans l'eau. Les solvants solubles ou miscibles dans l'eau comprennent les mono alcools à chaîne courte par exemple en $C_1$-$C_4$ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Selon une forme particulière de l'invention, les compositions de l'invention peuvent être anhydres.

Par "anhydre", on entend au sens de l'invention, une composition dont la teneur en eau libre ou ajoutée est inférieure à 3% et de préférence dont la teneur en eau ajoutée est inférieure à 1% en poids par rapport au poids total de la composition.

Les compositions selon l'invention peuvent contenir au moins une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones $D_4$, $D_5$ ou $D_6$.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Coming sous le nom de « Dow Coming 245 Fluid » ; les polyalkylarylsiloxanes comme par exemple les polyméthylphénylsiloxanes, commercialisés par la société Dow Coming sous le nom de « Dow Corning 556 Fluid » ; les copolymères polyéther et siloxane, comme par exemple les Diméthicone Copolyols.

Parmi les huiles émollientes non-volatiles utilisables dans la présente invention, on peut citer par exemple : les dérivés hydrocarbonés, les huiles minérales, les alcools gras, les esters d'alcools en $C_3$-$C_{18}$ avec des acides en $C_3$-$C_{18}$, les esters de l'acide benzoïque avec des alcools en $C_{12}$-$C_{18}$ et leurs mélanges, des polyols en $C_2$-$C_6$ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

Afin d'améliorer l'efficacité anti-transpirante de la composition, on peut utiliser en plus un ou plusieurs polymères anioniques hydrosolubles comprenant un acide Bronsted en particulier ceux dérivant de l'acide maléique et/ou de l'anhydride maléique qui sont décrits dans la demande de brevet WO02/49590.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une poudre organique.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 $\mu$m et masse volumique 40 kg/m3), 551 DE 20 (granulométrie d'environ 30 $\mu$m et masse volumique 65 kg/m3), 551 DE 50 (granulométrie d'environ 40 $\mu$m), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 $\mu$m et notamment allant de 0,02 $\mu$m à 1 $\mu$m, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges. La ou les poudres organiques peuvent être présentes par exemple en une quantité

Les compositions cosmétiques selon l'invention peuvent comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les épaississants peuvent également être cationiques comme par exemple le POLYQUATERNIUM-37 commercialisé sous la dénomination Salcare SC95 (Polyquaternium-37 (And) Mineral Oil (And) PPG-1 Trideceth-6) ou Salcare SC96 (Polyquaternium-37 (And) Propylene Glycol Dicaprylate/Dicaprate (And) PPG-1-Trideceth-6) ou d'autre polymère cationiques réticulés comme par exemple ceux de nom CTFA Copolymère Ethylacrylate / Dimethylamino Ethyl Methacrylate Cationique En Emulsion.

Les quantités de ces différents constituants pouvant être présents dans la composition cosmétique selon l'invention sont celles classiquement utilisées dans compositions déodorantes et/ou anti-transpirantes.

Lorsqu'elles contiennent une phase liquide organique non-miscible dans l'eau, les compositions selon l'invention lpeuvent également contenir également un ou plusieurs agents structurants ou gélifiants de ladite phase liquide organique non-miscible dans l'eau tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

Les compositions selon l'invention peuvent encore être pressurisées et être conditionnées dans un dispositif aérosol constitué par :

(A) un récipient comprenant une composition telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

Les exemples qui suivent servent à illustrer la présente invention.

### Exemple 1 : Roll on antitranspirant

| Matière première | teneur (% en poids) |
|---|---|
| ALUMINUM CHLOROHYDRATE | 20 |

(suite)

| Matière première | teneur (% en poids) |
|---|---|
| MICA (and) TITANIUM DIOXIDE (FLAMENCO ORANGE 320C -ENGELHARD)<br>Nacres : $d_{50}$ = 25$\mu$m<br>Taille max = 48$\mu$m | 0,7 |
| FRAGRANCE | 1 |
| DIMETHICONE | 0,50 |
| CETEARYL ALCOHOL | 2,50 |
| CETEARETH-33 | 1,25 |
| WATER | qsp 100 |

**<u>Exemple 2 : Aérosol antitranspirant</u>**

| Matière première | teneur (% en poids) |
|---|---|
| ALUMINUM CHLOROHYDRATE | 2 |
| STEARALKONIUM BENTONITE | 0,5 |
| ISOPROPYL PALMITATE | 2,5 |
| MICA (and) TITANIUM DIOXIDE (FLAMENCO ORANGE 320C - ENGELHARD) | 0,5 |
| FRAGRANCE | 1 |
| ISOBUTANE | 85 |
| CYCLOPENTASILOXANE (and) DIMETHICONOL | 0,5 |
| TRIETHYL CITRATE | 1 |
| CYCLOPENTASILOXANE | qsp 100 |

**<u>Mesure du pouvoir homogénéisant, pouvoir éclaircissant et des caractéristiques de transparence des exemples 1 et 2</u>**

**<u>Protocole</u>**

Dans un premiers temps, les compositions à analyser sont étalées de façon homogène à l'aide d'un applicateur de film de type ELCOMETER AUTOMATIC FILM APPLICATOR K4330 et un mobile standardisé à 50$\mu$m sur un transparent plastique standard. Chaque transparent est superposé à une plaque de contraste représentant un type de carnation de peau donnée. 4 zones $Z_1$, $Z_2$, $Z_3$ et $Z_4$ de carnation de peaux afro-américaines sont évaluées ainsi qu'une zone de référence $Z_0$. Des mesures colorimétriques L,a,b sont ensuite réalisées à l'aide d'un colorimétrique CHROMAMETER CR400® de MINOLTA sur chaque zone représentant chacune des carnations évaluées.

**<u>A) Pouvoir homogénéisant :</u>**

Le pouvoir homogénéisant est calculé à partir

- du $\Delta E_0$ moyen correspondant à la différence de couleur moyenne sur peau nue par rapport à la zone Zo de référence et

- du $\Delta$E moyen correspondant à la différence de couleur moyenne sur peau traitée par la composition de l'invention rapport à la zone Zo de référence.

Il se calcule par le rapport $\Delta E_0$ moyen/$\Delta$E moyen. Il est important que la composition présente une valeur de pouvoir homogénéisant supérieur à 1.5 pour qu'une bonne homogénéisation soit visible. Les résultats sont indiqués dans les tableaux ci-dessous :

**Pour l'exemple 1 :**

| Zones de carnation de la peau évaluées | Avant application | | | Après application | | |
|---|---|---|---|---|---|---|
| | **L** | **a** | **b** | **L** | **a** | **b** |
| $Z_0$ référence | 36.62 | 13.36 | 17.08 | 45.2 | 7.78 | 7.32 |
| $Z_1$ | 38.14 | 14.69 | 18 | 46.12 | 8.84 | 8.16 |
| $Z_2$ | 35.08 | 12.58 | 15.91 | 44.64 | 6.85 | 6.25 |
| $Z_3$ | 33.14 | 10.6 | 14.09 | 43.84 | 5.53 | 5.38 |
| $Z_4$ | 30.73 | 10.33 | 9.8 | 42.99 | 4.96 | 3.26 |

**Pour l'exemple 2 :**

| Zones de carnation de la peau évaluées | Avant application | | | Après application | | |
|---|---|---|---|---|---|---|
| | **L** | **a** | **b** | **L** | **a** | **b** |
| $Z_0$ référence | 36.62 | 13.36 | 17.08 | 55.2 | 7.38 | 11.31 |
| $Z_1$ | 38.14 | 14.69 | 18 | 55.77 | 7.88 | 11.62 |
| $Z_2$ | 35.08 | 12.58 | 15.91 | 54.53 | 6.92 | 10.77 |
| $Z_3$ | 33.14 | 10.6 | 14.09 | 54.37 | 6.2 | 10.27 |
| $Z_4$ | 30.73 | 10.33 | 9.8 | 53.17 | 6.03 | 9.15 |

| Composition testée | Support de l'exemple 1 : | Support de l'exemple 2 |
|---|---|---|
| Sans sel d'aluminium et sans nacres | 1,35 | 1,45 |
| Avec sel d'aluminium et sans nacres | 1,61 | 1,43 |
| Avec sel d'aluminium et sans nacres (invention) | 2,86 | 1,65 |

## B) Pouvoir éclaircissant :

Le pouvoir éclaircissant est le $\Delta$E moyen correspondant à la différence de couleur moyenne avant application de la composition et après application sur une zone de carnation de peau donnée Z. Il est important que la composition présente une valeur de pouvoir éclaircissant supérieur à 10 pour que l'effet soit réellement perceptible par le consommateur. Les résultats sont indiqués dans les tableaux ci-dessous :
Pour l'exemple 1 :

| Zone de carnation de la peau évaluée | Avant application | | | Après application | | |
|---|---|---|---|---|---|---|
| | **L** | **a** | **b** | **L** | **a** | **b** |
| Z | 36.62 | 13.36 | 17.08 | 45.2 | 7.78 | 7.32 |

**Pour l'exemple 2 :**

| Zone de carnation de la peau évaluée | Avant application | | | Après application | | |
|---|---|---|---|---|---|---|
| | L | a | b | L | a | b |
| Z | 36.62 | 13.36 | 17.08 | 55.2 | 7.38 | 11.31 |

| Composition testée | Support de l'exemple 1 : | Support de l'exemple 2 |
|---|---|---|
| Sans sel d'aluminium et sans nacres | 10,06 | 11,34 |
| Avec sel d'aluminium et sans nacres | 14,06 | 12,21 |
| Avec sel d'aluminium et **avec** nacres (invention) | 20,35 | 14,14 |

### C)Transparence :

Les valeurs de transparence sont quand à elles réalisées par l'intermédiaire de mesures colorimétriques en coordonnées X, Y, Z sur une plaque de contraste noir/blanc. La transparence est également un élément important puisque déterminant dans la perception de la formule par le consommateur. Il est préférable de conserver une transparence supérieure à 60% pour avoir un effet naturel.

Le pourcentage de transparence T est donnée par l'équation suivante :

$$T = 100 - ((Y \text{ zone noire} / Y \text{ zone blanche}) \times 100)$$

Les résultats sont indiqués dans le tableau ci-dessous :

| Composition testée | Support de l'exemple 1 : | Support de l'exemple 2 |
|---|---|---|
| Sans sel d'aluminium et sans nacres | 88,99 | 87,94 |
| Avec sel d'aluminium et sans nacres | 85,25 | 87,67 |
| Avec sel d'aluminium et **avec** nacres (invention) | 72,05 | 84,98 |

### Mise en avant de l'amélioration des différences de blanchiment/transferts sur les vêtements.

La technique mise en oeuvre permet ici de caractériser de façon statistiquement reproductible les effets de transferts et de blanchiment que peuvent occasionner l'utilisation des formules antitranspirantes.

Le principe de ce test est de filmer, dans un temps donné, l'évolution du blanchiment d'une formule lors de son application puis de mimer le transfert de celle-ci sur les vêtements.

Les formules sont appliquées sur des peaux ® Bioskin plates. Ce sont des plaques souples, constituées d'une double épaisseur de polyuréthane recouverte d'une fine couche de protection pour limiter la coloration et aider au démaquillage. Ce sont des sortes de « peaux synthétiques » qui ont pour but de reproduire l'élasticité, la souplesse de l'épiderme et la texture de la peau humaine.

Ce test se pratique à l'aide d'une camera (PIEPER GMBH 4912-5100) et d'un logiciel d'analyse d'images (Sedimet).

### Etape 1 : blanchiment intrinsèque de la formule

Cette étape nous permet d'évaluer le blanchiment de la formule après séchage.

1- on étale 400mg de formule sur une Bioskin noire sur une surface de 25 cm$^2$ (carré), avec le doigt

On filme pendant 3 heures dans la boite à lumière avec un intervalle de mesure de 5 minutes

## Etape 2 : blanchiment en « condition d'usage »

On essai ici de mimer ce qu'il se produit lors de l'application en appliquant un mouvement sur le support Pour cela, on casse le film de la bioskin en l'entourant autour d'un aérosol verre, prendre une photo dans la boite à lumière et on répète cette manipulation trois fois.

## Etape 3 : mesure du transfert sur tissu

On évalue le potentiel de la formule à tâcher les vêtements.

On dépose le tissu (jersey noir 100% coton) sur la ® Bioskin. On place un poids de 2Kg exactement sur la surface où la formule avait été déposée et on tire sur le tissu (placé entre la ® Bioskin et le poids) en entraînant le poids en même temps - le tirer vers le bas jusqu'à la fin de la bioskin puis on fait remonter le tissu jusqu'à son état initial. On prend une photo dans la boite à lumière du tissu.

On mesure le blanchiment en pourcentage de gris : 100% correspondant à un blanchiment total du tissu.

On compare la composition de l'exemple 1 avec une composition A identique mais dans laquelle les nacres selon l'invention sont remplacées par des nacres dont 50% de la population a un diamètre inférieur à 47$\mu$m et dont la population comporte des particules de taille de 128 $\mu$m.

| Exemple 1 (invention) | Exemple A (hors invention) |
|---|---|
| Nacres : $d_{50}$ = 25$\mu$m<br>Taille max = 48$\mu$m | Nacres : $d_{50}$ = 47$\mu$m<br>Taille max = 128 $\mu$m |
| 42.6 % | 47,5% |

On constate que le blanchiment du tissu est sensiblement plus important avec la composition A

### Exemple 3 : Stick antitranspirant

| Matière première | teneur (% en poids) |
|---|---|
| ALUMINIUM ZIRCONIUM TETRACHLOROHYDREX GLY | 20 |
| MICA (and) TITANIUM DIOXIDE (FLAMENCO ORANGE 320C ENGELHARD) | 0,5 |
| ISOPROPYL PALMITATE | 15 |
| HYDROGENATED CASTOR OIL | 3 |
| CYCLOPENTASILOXANE | 20 |
| POLYDECENE | 18 |
| PEG-8 DISTEARATE | 1,5 |
| STEARYL ALCOHOL | 17 |
| PARFUM | 1 |
| PPG-14 BUTYL EHTER | qsp 100 |

### Exemple 4 : Roll on antitranspirant hydro alcoolique

| Matière première | teneur (% en poids) |
|---|---|
| ALUMINIUM CHOLOROHYDRATE | 20 |
| MICA (and) TITANIUM DIOXIDE (FLAMENCO ORANGE 320C ENGELHARD) | 0,5 |
| ALCOOL DENATURE. | 40 |
| HYDROXYETHYLCELLULOSE | 0,5 |
| PARFUM | 1 |
| EAU | qsp 100 |

**Revendications**

1. Composition comprenant dans un milieu cosmétiquement acceptable

   a) au moins un actif anti-transpirant et
   b) au moins des particules interférentielles dont 50% de la population massique a un diamètre ($d_{50}$) inférieur à 40$\mu$m et dont la population massique ne comporte pas de particule de taille supérieure à 80 $\mu$m.

2. Composition selon la revendication 1, **caractérisée en ce que** 50% de la population massique a un diamètre ($d_{50}$) inférieur à 30 $\mu$m et plus particulièrement inférieur à 20 $\mu$m et en particulier inférieur à 15 $\mu$m.

3. Composition selon l'une quelconque des revendications précédentes, **caractérisée en ce que** les particules interférentielles sont choisies parmi les nacres et les pigments interférentiels.

4. Composition selon l'une quelconque des revendications précédentes, **caractérisée en ce que** les particules interférentielles comprennent au moins des nacres de type mica/oxyde d'étain/oxyde de titane et/ou de type mica/oxyde de fer/oxyde de titane.

5. Composition l'une quelconque des revendications précédentes, **caractérisée en ce que** les actifs anti-transpirants sont choisis parmi les sels d'aluminium et/ou de zirconium ; les complexes d'hydroxychlorure de zirconium et d'hydroxychlorure d'aluminium avec un acide aminé.

6. Composition selon la revendication 5, où l'actif anti-transpirant est le chlorhydrate d'aluminium sous forme activée ou non activée.

7. Composition selon l'une quelconque des revendications précédentes, **caractérisée en ce qu'**elle est conditionné dans un dispositif aérosol ou dans un flacon pompe ; dans un dispositif muni d'une paroi ajourée telle qu'une grille ; sous forme de bâtonnet.

8. Composition selon l'une quelconque des revendications précédentes, **caractérisée en ce qu'**elle est anhydre.

9. Composition selon l'une quelconque des revendications précédentes **caractérisée en ce qu'**elle contient au moins une phase liquide organique non-miscible dans l'eau.

10. Composition selon la revendication 9, où la phase liquide organique non-miscible dans l'eau contient une ou plusieurs huiles émollientes siliconée ou hydrocarbonée, volatiles ou non-volatiles.

11. Composition selon l'une quelconque des revendications 1 à 10, contenant en plus au moins un additif choisi parmi les polymères anioniques hydrosolubles comprenant un acide Bronsted, les agents de suspension, les poudres organiques.

12. Procédé cosmétique de maquillage en particulier de la peau des aisselles et de traitement de la transpiration, consistant à appliquer sur la surface de la peau une quantité efficace d'une composition telle que définie dans les revendications 1 à 11.

13. Procédé cosmétique de maquillage en particulier de la peau des aisselles et de traitement des odeurs corporelles en particulier des odeurs axillaires, consistant à appliquer sur la surface de la peau une quantité efficace d'une composition telle que définie dans les revendications 1 à 11.

14. Utilisation de particules interférentielles dont 50% de la population massique a un diamètre ($d_{50}$) inférieur à 40$\mu$m et dont la population massique ne comporte pas de particule de taille supérieure à 80 $\mu$m telles que définies dans les revendications précédentes dans une composition comprenant dans un milieu cosmétiquement acceptable au moins un actif anti-transpirant dans le but de masquer les irrégularités de la peau en particulier de la peau des aisselles après application de ladite composition.

**Claims**

1. Composition comprising, in a cosmetically acceptable medium:

   a) at least one antiperspirant active principle
   and
   b) at least interference particles for which 50% of the population by weight has a diameter ($d_{50}$) of less than 40 $\mu$m and for which the population by weight does not comprise particles with a size of greater than 80 $\mu$m.

2. Composition according to Claim 1, **characterized in that** 50% of the population by weight has a diameter ($d_{50}$) of less than 30 $\mu$m and more particularly of less than 20 $\mu$m and in particular of less than 15 $\mu$m.

3. Composition according to either one of the preceding claim, **characterized in that** the interference particles are chosen from pearlescent agents and interference pigments.

4. Composition according to any one of the preceding claims, **characterized in that** the interference particles comprise at least pearlescent agents of mica/tin oxide/titanium oxide type and/or of mica/iron oxide/titanium oxide type.

5. Composition according to any one of the preceding claims, **characterized in that** the antiperspirant active principles are chosen from aluminium and/or zirconium salts; or complexes of zirconium hydroxychloride and of aluminium hydroxychloride with an amino acid.

6. Composition according to Claim 5, where the antiperspirant active principle is aluminium chlorohydrate, in the activated or nonactivated form.

7. Composition according to any one of the preceding claims, **characterized in that** it is packaged in an aerosol device or in a pump-action spray; in a device provided with an openwork wall, such as a grating; or in the form of a stick.

8. Composition according to any one of the preceding claims, **characterized in that** it is anhydrous.

9. Composition according to any one of the preceding claims, **characterized in that** it comprises at least one water-immiscible organic liquid phase.

10. Composition according to Claim 9, where the water-immiscible organic liquid phase comprises one or more volatile or nonvolatile silicone or hydrocarbon emollient oils.

11. Composition according to any one of Claims 1 to 10, additionally comprising at least one additive chosen from water-soluble anionic polymers comprising a Bronsted acid, suspending agents and organic powders.

12. Cosmetic method for making up in particular the skin of the armpits and for treating perspiration which consists in applying, to the surface of the skin, an effective amount of a composition as defined in Claims 1 to 11.

13. Cosmetic method for making up in particular the skin of the armpits and for treating body odours, in particular axillary odours, which consists in applying, to the surface of the skin, an effective amount of a composition as defined in Claims 1 to 11.

14. Use of interference particles for which 50% of the population by weight has a diameter ($d_{50}$) of less than 40 $\mu$m and for which the population by weight does not comprise particles with a size of greater than 80 $\mu$m as defined in the preceding claims in a composition comprising, in a cosmetically acceptable medium, at least one antiperspirant active principle for the purpose of concealing the irregularities of the skin, in particular of the skin of the armpits, after application of the said composition.


**Patentansprüche**

1. Zusammensetzung, umfassend in einem kosmetisch unbedenklichen Medium:

   a) mindestens ein schweißhemmendes Mittel und

b) mindestens Interferenzteilchen, wobei 50% der gewichtsbezogenen Population einen Durchmesser ($d_{50}$) von weniger als 40 $\mu$m aufweisen und die gewichtsbezogene Population keine Teilchen mit einer Größe von mehr als 80 $\mu$m umfasst.

2. Zusammensetzung nach Anspruch 1, **dadurch gekennzeichnet, dass** 50% der gewichtsbezogenen Population einen Durchmesser ($d_{50}$) von weniger als 30 $\mu$m und spezieller weniger als 20 $\mu$m und speziell weniger als 15 $\mu$m aufweisen.

3. Zusammensetzung nach einem der vorhergehenden Ansprüche, **dadurch gekennzeichnet, dass** die Interferenzteilchen unter Perlglanzmitteln und Interferenzpigmenten ausgewählt sind.

4. Zusammensetzung nach einem der vorhergehenden Ansprüche, **dadurch gekennzeichnet, dass** die Interferenzteilchen mindestens Perlglanzmittel vom Typ Glimmer/Zinnoxid/Titanoxid und/oder vom Typ Glimmer/Eisenoxid/Titanoxid umfassen.

5. Zusammensetzung nach einem der vorhergehenden Ansprüche, **dadurch gekennzeichnet, dass** die schweißhemmenden Mittel unter Aluminium- und/oder Zirconiumsalzen und Komplexen von Zirconiumhydroxidchlorid und Aluminiumhydroxidchlorid mit einer Aminosäure ausgewählt sind.

6. Zusammensetzung nach Anspruch 5, wobei es sich bei dem schweißhemmenden Wirkstoff um Aluminiumhydroxidchlorid in aktivierter oder nicht aktivierter Form handelt.

7. Zusammensetzung nach einem der vorhergehenden Ansprüche, **dadurch gekennzeichnet, dass** sie in einer Aerosolvorrichtung oder in einer Pumpflasche, in einer Vorrichtung mit einer durchbrochenen Wand wie einem Gitter oder in Form eines Stifts konfektioniert ist.

8. Zusammensetzung nach einem der vorhergehenden Ansprüche, **dadurch gekennzeichnet, dass** sie wasserfrei ist.

9. Zusammensetzung nach einem der vorhergehenden Ansprüche, **dadurch gekennzeichnet, dass** sie mindestens eine nicht wassermischbare organische flüssige Phase enthält.

10. Zusammensetzung nach Anspruch 9, wobei die nicht wassermischbare organische flüssige Phase ein oder mehrere flüchtige oder nichtflüchtige Silikon- oder Kohlenwasserstoff-Emolliensöle enthält.

11. Zusammensetzung nach einem der Ansprüche 1 bis 10, die außerdem mindestens ein Additiv, das unter wasserlöslichen anionischen Polymeren, die eine Brönsted-Säure umfassen, Suspendiermitteln und organischen Pulvern ausgewählt ist, enthält.

12. Kosmetisches Verfahren zum Überdecken insbesondere der Achselhaut und zur Behandlung von Schweißabsonderung, das darin besteht, dass man auf die Oberfläche der Haut eine wirksame Menge einer Zusammensetzung gemäß einem der Ansprüche 1 bis 11 aufbringt.

13. Kosmetisches Verfahren zum Überdecken insbesondere der Achselhaut und zur Behandlung von Körpergerüchen, insbesondere Achselgerüchen, das darin besteht, dass man auf die Oberfläche der Haut eine wirksame Menge einer Zusammensetzung gemäß einem der Ansprüche 1 bis 11 aufbringt.

14. Verwendung von Interferenzteilchen, wobei 50% der gewichtsbezogenen Population einen Durchmesser ($d_{50}$) von weniger als 40 $\mu$m aufweisen und die gewichtsbezogene Population keine Teilchen mit einer Größe von mehr als 80 $\mu$m umfasst, gemäß einem der vorhergehenden Ansprüche in einer Zusammensetzung, die in einem kosmetisch unbedenklichen Medium mindestens ein schweißhemmendes Mittel umfasst, zur Maskierung von Unregelmäßigkeiten der Haut, insbesondere der Achselhaut, nach dem Aufbringen der Zusammensetzung.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2000086446 A
- EP 972512 A
- US 3792068 A
- US 4822596 A
- US 4904463 A
- WO 9744010 A

**Littérature non-brevet citée dans la description**

- **C. FOX.** *Cosmetics and Toiletries,* Novembre 1986, vol. 101, 101-112